# EUROPEAN PATENT SPECIFICATION

(11) **EP 1 313 413 B1**
(45) Date of publication and mention of the grant of the patent: **29.12.2004**
(21) Application number: 01960973.4
(22) Date of filing: 29.08.2001
(51) Int. Cl.: A61F 5/37

(54) **SOFT RESTRAINT SYSTEM**
WEICHES RÜCKHALTESYSTEM
SYSTEME SOUPLE DESTINE A MAITRISER UN INDIVIDU TURBULENT

(30) Priority: 29.08.2000 GB 0021177
(43) Date of publication of application: 28.05.2003
(73) Proprietor: Deenside Limited, Northampton NN1 4JE (GB); Hickling, Ruth Marjery HF, Northampton NN1 4JE (GB)
(72) Inventor: JONES, Philip David, Isle of Wight PO32 6AH (GB); HICKLING, Alan DI, (GB)
(74) Representative: Gregory, Timothy Mark
(86) International application number: PCT/GB2001/003851
(87) International publication number: WO 2002/017829

(56) References cited:
- US-A- 2 664 083
- US-A- 4 571 000
- US-A- 4 852 587
- US-A- 5 031 639
- US-A- 5 111 850

## Description

The present invention relates to a soft restraint system. More particularly, but not exclusively, it relates to a soft restraint system enabling people upholding the law and other responsible personnel to restrain a troublemaker or miscreant engaged in disorderly or criminal behaviour.

In one example it may be used on board aircraft. The term 'air rage' has been used in recent times to describe airline passengers who, for whatever reason, become disruptive or violent whilst an aircraft is in flight. In so doing, they may annoy fellow passengers, or may even disrupt or endanger the flight. This has caused airline companies considerable concern. Indeed, on occasions, aircraft have been diverted to unload the miscreant.

The soft restraint system may of course be used physically to restrain miscreants or other troublesome individuals in many other situations, such as by police, prison officers, nurses or other personnel engaged in law enforcement operations, or in any other situation where people are likely to be faced with a person out of control or likely to do violence either to himself, herself or those around him or her, such as in psychiatric hospitals, etc.

One increasing problem these days is that often, the staff most immediately available to deal with a miscreant are female or people less strong than the person who must be restrained.

At present, it is known to use a restraining set of handcuffs, but these are not easy to apply and a certain amount of force is needed to bring the miscreant's hands together before the handcuffs can be applied.

It is also known from our co-pending UK Patent Application No. 9927364.1 to provide a restraint system which includes a tubular member adapted to be placed loosely over the head and around the torso of the miscreant. However, this restraint system then needs to be held in place by means of padlocks, firstly to narrow the neck opening of the tubular member and secondly to lock the lower end of the tubular member between the legs of the miscreant. By use of this device, the miscreant can be rendered harmless since the hands and any weapons carried thereby will be enclosed within the tubular member.

A device according to the first part of Claim 1 is known from US-A-2 664 063.

However, the member is not always easy to apply, particularly if the miscreant does not wish it to be applied. Finding and applying the padlocks can take valuable seconds. Furthermore, the shape of the restraint system sometimes makes it difficult or awkward to apply the system over the head and shoulders of a miscreant.

It is an object of the invention to provide a system which overcomes the above disadvantages and which enables application of a first degree of restraint to the miscreant, i.e. to make it difficult but not impossible for him to react. Furthermore, it should enable application of a second degree of restraint to be applied immediately or soon thereafter so that the miscreant is restrained from further damage to himself, herself or those around him or her.

According to a first aspect of the present invention, there is provided a device for restraining a miscreant, said device comprising a substantially tubular body adapted to envelop the torso of a miscreant and having an upper, in use, opening adapted to be passed over the head to surround the neck of the miscreant, and a lower, in use, opening, said body being provided with a restraint strap system which comprises a pair of straps each adapted to encircle substantially one half of the tubular body and locking means for each end of each of said straps disposed at two substantially diametrically opposed locations on the tubular body.

Preferably, said straps so pass through the locking means as to be manually graspable at either free end, whereby manual traction of either free end of a strap will cause lockable tightening of that strap around part of the body.

Advantageously, the restraint strap system comprises two pairs of straps, one disposed above, in use, the other.

In this case, the upper, in use, pair of straps is adapted to encircle the miscreant in the region of his elbows and the lower, in use, pair of straps is adapted to encircle the miscreant in the region of his knees.

The body preferably has a substantially frustoconical shape extending from a comparatively narrow upper, in use, end to a wider lower, in use, end.

The substantially frustoconical shape may be described as a bell-shape, in which case a wide, lowermost end is adapted more easily to envelop the head and shoulders of the miscreant.

Also narrowing of the bell-shape towards an upper end of the body is adapted to constrict the shoulders and/or upper arms of the miscreant as the body of the device is pulled downwardly over the torso.

The material of the body is preferably of a foraminous woven material.

The locking means may comprise a pair of D-rings at each end of each strap.

According to a second aspect of the present invention, there is provided a method of restraining a miscreant comprising the steps of providing a device as described above, deploying said device generally above the head of said miscreant, pulling the device downwardly over the head and torso of the miscreant until the opening at the upper end of the tubular body has passed the head of the miscreant, and manually pulling one free end of each strap means to cause lockable tightening thereof around the body of the device, thereby restraining the miscreant at at least one location.

In the case where there are two strap systems, it is preferred that the upper, in use, strap system be tightened prior to tightening the lower, in use, strap system.

An embodiment of the present invention will now be more particularly described by way of example and with reference to the accompanying drawings in which:
Figure 1 is a front elevation of a device embodying the invention;
Figure 2 is a rear elevation of the device shown in Figure 1;
Figure 3 is a scrap view of locking means for the device when in a relaxed condition to enable unlocking;
Figure 4 is a scrap view of the locking means when the straps are locked; and
Figure 5 shows an alleged miscreant suitably restrained by the device.

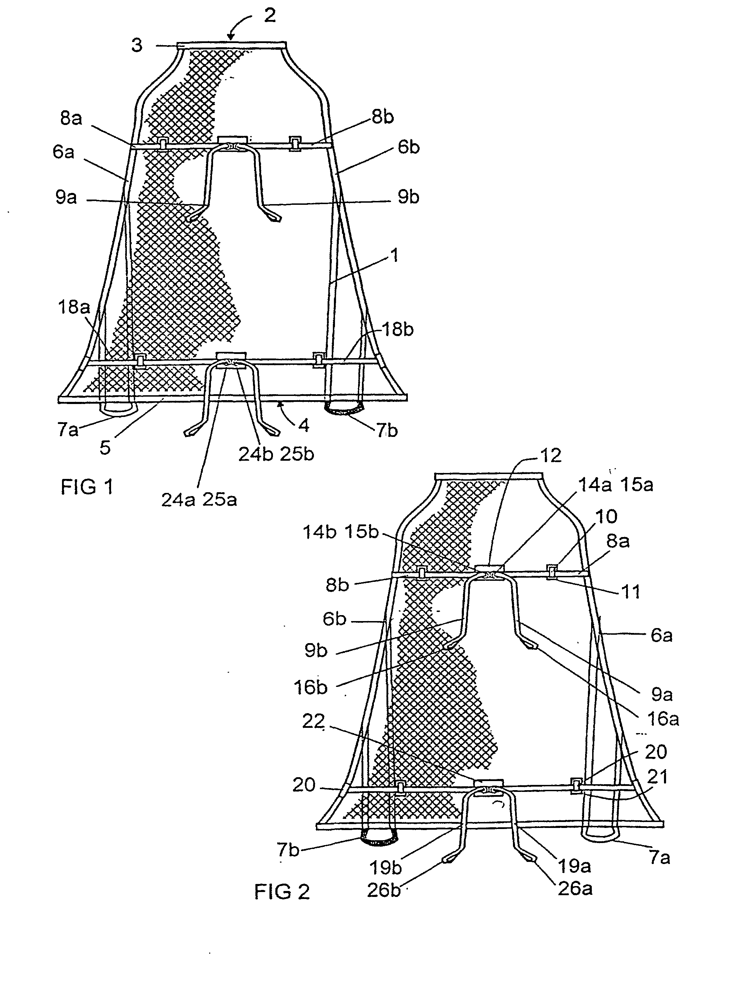

Referring now to the drawings, more particularly to Figures 1 and 2, there is shown a restraint device which comprises a substantially bell-shaped body 1 of foraminous woven material generally dimensioned to encircle the torso of a miscreant of at least average size. The device may be produced in a range of sizes to suit the problem. The body 1 is formed of two pieces of material joined at side seams 6a and 6b. At its upper, in use, end is an opening 2, defined by a substantially inextendible seam 3, dimensioned to fit over the head of the miscreant and encircle his neck. The lower, in use, end of the body 1 is of much greater circumference so as more easily to encapsulate the head and shoulders of the miscreant, in a manner which will be described below. The lower opening 5 is encircled by a seam 4 of substantially inextendible material and is provided with two pairs of handles, 7a and 7b. These handles 7a and 7b extend to the side seams 6a and 6b respectively and are affixed to the body and to the lower and the side seams by stitching or the like along their length. This prevents tearing of the foraminous material when force is applied to the handles.

In order to carry out a first phase of restraint of a miscreant, an empowered operative, who may be a police officer, a prison warder, a member of airline personnel, a nurse or the like should have the device to hand. It may possibly be so folded into a belt-mounted bag that it may be deployed at a moment's notice. Preferably, the handles 7a and 7b protrude from the bag or are easily available for use by a pair of operatives. The handles may be so colour coded or otherwise identifiable that one operative may take the pair of handles 7a and the other may take the pair of handles 7b. The two operatives then place the bell mouth lower opening 5 above the head of the miscreant and bring the device 1 down sharply so that it envelops first the head and then the torso of the miscreant, with the handles 7a and 7b generally finishing in the calf region of the miscreant.

The upper opening 2 will at that point surround the neck of the miscreant. The upper part: of the bell-shape of the device 1 will tend, as it is pulled downwardly, to constrict the shoulders and upper arms of the miscreant so that it is more difficult, but not yet impossible, for the miscreant to deploy his arms aggressively.

At this point, it is important to move on to the second stage of restraint as soon as possible. For this, there are provided two strap systems:-
upper, in use, straps 8a and 8b, are located to encircle the miscreant in the region just above the elbows; and
lower, in use, straps 18a and 18b, are located to encircle the miscreant in the region just above the knees.

The upper strap system comprises two straps 8a and 8b, each passing around a respective side of the body 1 from substantially the middle of the front to substantially the middle of the back face of the body 1. The straps 8a and 8b pass through loops 10, each fixed to a reinforcement patch 11, themselves attached to the material of the body 1. As shown, there are at least two loops 10 for each strap 8a,8b (one at the front and one at the rear), although more may be provided if so desired.

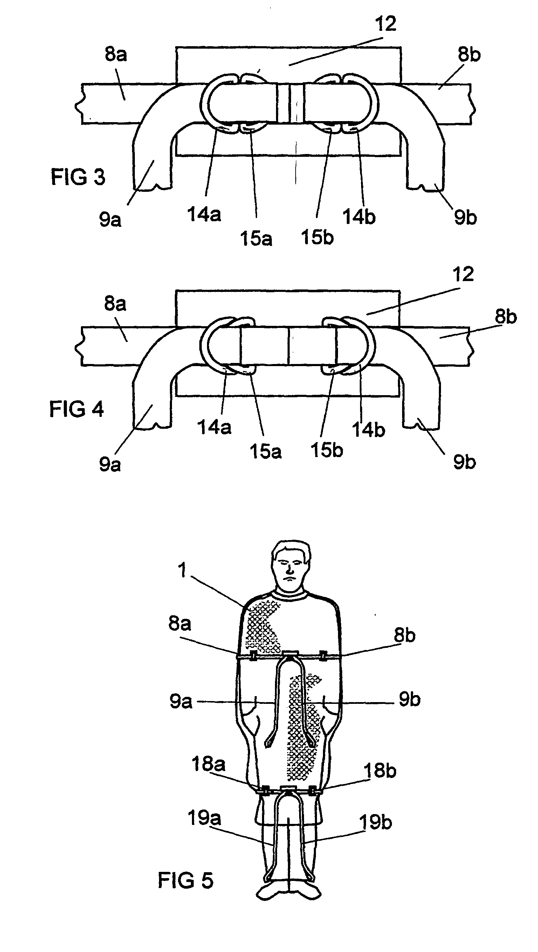

At the centre of both front and rear panels of the body 1 there is provided a reinforcement patch 12 to which are mounted pairs of locking D-rings 14a and 15a, 14b and 15b. As shown in Figures 3 and 4, each end of each strap 8 passes through an upper D-ring 14 and then through and around a lower D-ring 15, passing back through the upper D-ring 14 and ending in a grip 9a or 9b. The end of each grip 9 is folded over on itself and sewn at 16a,16b.

Figure 3 shows this arrangement in relaxed condition where a strap may be released by passing the strap 8 through the upper D-ring 14, toward the centre of the reinforcement patch 12.

Figure 4 shows the arrangement when the grips 9a and 9b have been pulled outwardly to tighten the straps 8a and 8b. The D-ring 15 is flipped over to trap the strap 8 between it and D-ring 14, thereby causing the strap to become locked. It can be further tightened by pulling the grip 9a, but cannot be released without moving D-ring 15 back to its position as shown in Figure 3. To this end, each D-ring 15 may optionally be provided with a tag or pull-string to facilitate the release process.

As can be seen, there are two pairs of grips 9a,9b - one pair to the front of the body 1 and one pair to the rear. Pulling on either grip will cause tightening of the corresponding strap 8 around the respective side of the miscreant. The other end of that strap will be or become locked by the D-rings at the other side of the body 1.

Either hand of each operative may be used to pull the grip at either the front or rear of the respective side of the miscreant. Ideally, both operatives will pull the grips on the same side of the body 1, since this gives a more controlled action with regard to the miscreant. However, when urgent action is required, it may be necessary for one operative to pull his rear grip and the other to pull his front grip. Indeed, it may be possible for a single operative to pull both grips 9a and 9b of one pair, one away from the other.

The lower strap system includes two straps 18a, 18b, each passing around a respective side of the body, from the middle of the front to the middle of its back. The straps pass through loops 20 stitched to reinforcement patches 21 and again pass through pairs of D-rings 24a, 25a and 24b and 25b, all stitched or attached to reinforcement patch 22. The loose ends of the straps 18a and 18b form respectively pulling grips 19a and 19b. Again, if each operative pulls one of the grips 19a or 19b, the straps will become tightened and locked so that the knees of the miscreant will be restrained and he will be finally unable to make nuisance of himself. This situation is shown in Figure 5.

As has been described, the system is especially suitable for use by operatives less strong than the miscreant since they can pull downwardly against the individual's struggles upwardly. In such a contest, the downward force usually wins.

The device of the invention is generally safe to use as it avoids restraint techniques associated with medical problems such as positional asphyxia and it is made of foraminous breathable material. It is also lightweight and therefore the miscreant should not suffer from overheating or be forced to carry a heavy burden. Hence, it can be retained in position for lengthy periods of time until the miscreant can be handed over to the police or be dealt with by more permanent forms of restraint, if that is appropriate.

The apparatus embodying the invention is very easy to use, and does not involve intensive training. It can be applied quickly from a stowage container which can be carried by the person of an operative or may be located at convenient points within any space where trouble could be expected.

The device may be used in aeroplanes, in police cells, in prisons, in hospitals and in arrest situations and indeed anywhere where a miscreant may be breaking the law or causing civil unrest.

## Claims

1. A device for restraining a miscreant, said device comprising a substantially tubular body (1) adapted to envelop the torso of a miscreant and having an upper, in use, opening (2) adapted to be passed over the head to surround the neck of the miscreant, and a lower, in use, opening (5), said body (1) being provided with a restraint strap system (8a, 8b, 18a, 18b) which comprises at least one pair of straps **characterised in that** each strap is adapted to encircle substantially one half of the tubular body (1) and **in that** said restraint strap system comprises locking means (14a, 14b, 15a, 15b, 24a, 24b, 25a, 25b) for each end of each of said straps disposed at two substantially diametrically opposed locations on the tubular body.

2. A device according to claim 1, **characterised in that** the straps so pass through the locking means as to be manually graspable at either free end (9a, 9b), whereby manual traction of either free end of a strap (9a, 9b) will cause lockable tightening of that strap around part of the body.

3. A device according to either claim 1 or claim 2, **characterised in that** the restraint strap system comprises two pairs of straps (8a, 8b, 18a, 18b), one disposed above, in use, the other.

4. A device according to claim 3, **characterised in that** the upper, in use, pair of straps (8a, 8b) is adapted to encircle the miscreant in the region of his elbows and the lower, in use, pair of straps (18a, 18b) is adapted to encircle the miscreant in the region of his knees.

5. A device according to any one of the preceding claims, **characterised in that** the body (1) has a substantially frustoconical shape extending from a comparatively narrow upper, in use, end to a wider lower, in use, end.

6. A device according to any one of the preceding claims, **characterised in that** the material of the body comprises a foraminous woven material.

7. A device according to any one of the preceding claims, **characterised in that** the locking means comprises a pair of D-rings (14a, 14b, 15a, 15b) at each end of each strap.

8. A method of restraining a miscreant comprising the steps of providing a device according to any one of the preceding claims, deploying said device (1) generally above the head of said miscreant, pulling the device downwardly over the head and torso of the miscreant until the opening at the upper end of the tubular body has passed the head of the miscreant, **characterised by** the steps of manually pulling one free end (9a, 9b) of each strap means (8a, 8b, 18a, 18b) to cause lockable tightening thereof around the body of the device, thereby restraining the miscreant at at least one location.

9. A method according to claim 8, when dependant on any one of claims 3 to 7 where there are two strap systems (8a, 8b, 18a, 18b), **characterised by** the further step of tightening the upper, in use, strap system prior to tightening the lower, in use, strap system.

## Patentansprüche

1. Vorrichtung zum Festhalten eines Straftäters, wobei die Vorrichtung einen im Wesentlichen röhrenförmigen Körper (1) umfasst, der dafür ausgelegt ist, den Rumpf eines Straftäters zu umgeben, die Vorrichtung bei Gebrauch eine obere Öffnung (2) aufweist, die dafür ausgelegt ist, über den Kopf gestülpt zu werden, um den Hals des Straftäters zu umgeben, und die Vorrichtung bei Gebrauch eine untere Öffnung (5) aufweist, wobei der Körper (1) mit einem Halteriemensystem (8a, 8b, 18a, 18b) versehen ist, das mindestens ein Paar Riemen umfasst, **dadurch gekennzeichnet, dass** jeder Riemen dafür ausgelegt ist, im Wesentlichen eine Hälfte des röhrenförmigen Körpers (1) zu umgeben, und dass das Halteriemensystem Verriegelungsmittel (14a, 14b, 15a, 15b, 24a, 24b, 25a, 25b) für jedes Ende jedes der Riemen umfasst, die an zwei im Wesentlichen diametral gegenüberliegenden Positionen am röhrenförmigen Körper angeordnet sind.

2. Vorrichtung nach Anspruch 1, **dadurch gekennzeichnet, dass** die Riemen so durch das Verriegelungsmittel geführt werden, dass sie an beiden freien Enden (9a, 9b) manuell gegriffen werden können, wobei ein manuelles Ziehen an beiden freien Enden eines Riemens (9a, 9b) ein verriegelungsfähiges Strammziehen dieses Riemens um einen Teil des Körpers verursacht.

3. Vorrichtung nach Anspruch 1 oder 2, **dadurch gekennzeichnet, dass** das Halteriemensystem zwei Paare Riemen (8a, 8b, 18a, 18b) umfasst, wobei bei Gebrauch einer über dem anderen angeordnet ist.

4. Vorrichtung nach Anspruch 3, **dadurch gekennzeichnet, dass** das obere Riemenpaar (8a, 8b) bei Gebrauch dafür ausgelegt ist, den Straftäter im Bereich seiner Ellbogen zu umgeben, und das untere Riemenpaar (18a, 18b) bei Gebrauch dafür ausgelegt ist, den Straftäter im Bereich seiner Knie zu umgeben.

5. Vorrichtung nach einem der vorhergehenden Ansprüche, **dadurch gekennzeichnet, dass** der Körper (1) eine im Wesentlichen kegelstumpfförmige Form aufweist, die sich von einem bei Gebrauch vergleichsweise engen oberen Ende in ein bei Gebrauch breiteres unteres Ende erweitert.

6. Vorrichtung nach einem der vorhergehenden Ansprüche, **dadurch gekennzeichnet, dass** das Material des Körpers ein mit Löchern versehenes gewobenes Material umfasst.

7. Vorrichtung nach einem der vorhergehenden Ansprüche, **dadurch gekennzeichnet, dass** das Verriegelungsmittel ein Paar D-Ringe (14a, 14b, 15a, 15b) an jedem Ende jedes Riemens umfasst.

8. Verfahren zum Festhalten eines Straftäters, das folgende Schritte umfasst: Bereitstellen einer Vorrichtung nach einem der vorhergehenden Ansprüche, Anbringen der Vorrichtung (1) im Allgemeinen über dem Kopf des Straftäters, Ziehen der Vorrichtung nach unten über den Kopf und den Rumpf des Straftäters, bis die Öffnung am oberen Ende des röhrenförmigen Körpers über den Kopf des Straftäters gestülpt ist, **gekennzeichnet durch** folgende Schritte: manuelles Ziehen eines freien Endes (9a, 9b) jedes Riemenmittels (8a, 8b, 18a, 18b), um ein verriegelungsfähiges Strammziehen desselben um den Körper der Vorrichtung herum zu verursachen, wodurch der Straftäter in mindestens einer Position festgehalten wird.

9. Verfahren nach Anspruch 8, wenn es von einem der Ansprüche 3 bis 7 abhängig ist, wobei zwei Riemensysteme (8a, 8b, 18a, 18b) vorhanden sind, **gekennzeichnet durch** den weiteren Schritt des Strammziehens des oberen Riemensystems bei Gebrauch vor dem Strammziehen des unteren Riemensystems bei Gebrauch.

## Revendications

1. Appareil destiné à maîtriser un individu perturbateur, ledit appareil comprenant un corps essentiellement tubulaire (1) adapté pour envelopper le torse d'un individu perturbateur et ayant une ouverture supérieure (2), lors de son utilisation, adaptée pour être passée par-dessus la tête afin d'entourer le cou de l'individu perturbateur, et une ouverture inférieure (5), prête à l'emploi, ledit corps (1) étant fourni avec un système d'immobilisation par sangles (8a, 8b, 18a, 18b) qui comprend au moins une paire de sangles, **caractérisé en ce que** chaque sangle est adaptée pour entourer essentiellement une moitié du corps tubulaire (1) et **en ce que** ledit système d'immobilisation par sangles comprend un moyen de verrouillage (14a, 14b, 15a, 15b, 24a, 24b, 25a, 25b) pour chaque extrémité de chacune desdites sangles en deux endroits essentiellement diamétralement opposés sur le corps tubulaire.

2. Appareil selon la revendication 1, **caractérisé en ce que** les sangles passent à travers le moyen de verrouillage de façon à être saisissables à la main par l'une ou l'autre extrémité libre (9a, 9b), au moyen duquel la traction manuelle de l'une ou l'autre extrémité libre (9a, 9b) d'une sangle provoquera le serrage par verrouillage de cette sangle autour d'une partie du corps.

3. Appareil selon l'une quelconque des revendications 1 ou 2, **caractérisé en ce que** le système d'immobilisation par sangles comprend deux paires de sangles (8a, 8b, 18a, 18b), l'une étant disposée, lors de son utilisation, au-dessus de l'autre.

4. Appareil selon la revendication 3, **caractérisé en ce que** la paire supérieure de sangles (8a, 8b), lors de son utilisation, est adaptée pour entourer l'individu perturbateur dans la région de ses coudes et la paire inférieure de sangles (18a, 18b), lors de son utilisation, est adaptée pour entourer l'individu perturbateur dans la région de ses genoux.

5. Appareil selon l'une quelconque des revendications précédentes, **caractérisé en ce que** le corps (1) a une forme essentiellement tronconique qui s'étend d'une extrémité supérieure, lors de son utilisation, comparativement petite à une extrémité inférieure, lors de son utilisation, plus large.

6. Appareil selon l'une quelconque des revendications précédentes, **caractérisé en ce que** le matériau du corps comprend un matériau tissé perméable.

7. Appareil selon l'une quelconque des revendications précédentes, **caractérisé en ce que** le moyen de verrouillage comprend une paire d'anneaux en forme de D (14a, 14b, 15a, 15b) située à chaque extrémité de chaque sangle.

8. Procédé de maîtrise d'un individu perturbateur comprenant les étapes consistant à fournir un appareil selon l'une quelconque des revendications précédentes, déployer ledit appareil (1) en particulier par-dessus la tête dudit individu perturbateur, tirer l'appareil vers le bas par-dessus la tête et le torse de l'individu perturbateur jusqu'à ce que l'ouverture au niveau de l'extrémité supérieure du corps tubulaire ait passé la tête de l'individu perturbateur, **caractérisé par** les étapes consistant à tirer manuellement une extrémité libre (9a, 9b) de chaque sangle (8a, 8b, 18 a, 18b) dans le but de provoquer le serrage par verrouillage de celle-ci autour du corps de l'appareil, maîtrisant de cette façon l'individu perturbateur en au moins un endroit.

9. Procédé selon la revendication 8, lorsque dépendant de l'une quelconque des revendications 3 à 7 où sont localisés deux systèmes de sangles (8a, 8b, 18a, 18b), **caractérisé par** une étape supplémentaire consistant à serrer le système supérieur de sangles, lors de son utilisation, avant de serrer le système inférieur de sangles, lors de son utilisation.
